# EUROPÄISCHE PATENTANMELDUNG

(11) **EP 2 366 927 A1**
(43) Veröffentlichungstag der Anmeldung: **21.09.2011**
(21) Anmeldenummer: 10156519.0
(22) Anmeldetag: 15.03.2010
(51) Int. Cl.: F16L 3/22, A61M 5/14

(54) **Klemme und Klemmensystem sowie deren Verwendung**

(71) Anmelder: Fresenius Kabi Deutschland GmbH, 61352 Bad Homburg (DE)
(72) Erfinder: Lehmann, Björn, 36251 Bad Hersfeld (DE); Roser, Steffen, 61118 Bad Vilbel (DE); Pütter, Harry, 36364 Bad Salzschlirf (DE)
(74) Vertreter: Richly, Erik

(57) **Zusammenfassung**

Die Erfindung betrifft eine Klemme zur Aufnahme von zylindrischen Gegenständen mit einem Grundkörper (2), der an zwei einander gegenüberliegenden Seiten aufeinander abgestimmte Verbindungselemente (5, 6) zur lösbaren Verbindung gleichartiger Klemmen (1, 10, 11, 12) aufweist, und einem Aufnahmebereich (3), der zur lösbaren Rastverbindung eines zylindrischen Gegenstands (13) an dem Grundkörper (2) im Wesentlichen ringsegmentartig und mit einer Aufnahmeöffnung (8) ausgebildet ist, die sich auf weniger als 180° des Aufnahmebereichs (3) erstreckt, wobei der Innendurchmesser (D) des Aufnahmebereichs (3) 32 mm, 17 mm bis 24 mm oder 10 mm beträgt. Des Weiteren werden Systeme und Kombinationen umfassend die erfindungsgemäße Klemme offenbart.

## Beschreibung

Die Erfindung betrifft eine Klemme zur Aufnahme zylindrischer Gegenstände mit einem Grundkörper, der Verbindungselemente zur lösbaren Verbindung mit gleichartigen Klemmen aufweist, und mit einem Aufnahmebereich zur lösbaren Verbindung eines zylindrischen Gegenstands mit dem Grundkörper. Weiter betrifft die Erfindung ein System aus wenigstens zwei Klemmen, die Kombination aus einem solchen System und einer Hahnbank sowie die Verwendung einer Klemme bzw. eines Systems aus mehreren Klemmen zur Aufnahme von Spritzen.

Klemmen der eingangs genannten Art sind z. B. aus der DE 295 03 957 U1 bekannt. Diese aus dem Stand der Technik bekannten Klemmen werden zur Befestigung von Rohrleitungen oder Kabeln insbesondere in Gebäuden verwendet. In der Regel sind derartigen Klemmen allenfalls zur Verbindung mit identischen Klemmen, teilweise unter Verwendung zusätzlicher Verbindungsmittel, geeignet. Es ist damit nicht möglich, Rohre unterschiedlicher Querschnitte durch miteinander verbindbare Klemmen eines einheitlichen Systems zu befestigen.

Im Bereich der Medizin ist es bekannt, dass für einige Anwendungszwecke Arzneimittel nacheinander verabreicht werden müssen oder Komponenten einer nicht lagerstabilen Mischung von Arzneimitteln erst kurz vor der Anwendung miteinander gemischt werden dürfen. Üblicherweise werden die verschiedenen Arzneimittel jeweils mit einer Spritze über einen Zugangsport in den Infusionsbeutel zudosiert. Da die Medikamente in der Regel in Apotheken zur Verfügung gestellt werden, besteht die Gefahr, dass die einzelnen Komponenten einer Mischung während des Transports oder der Lagerung verwechselt werden und eine sichere Zuordnung erschwert ist. Auch die teilweise relevante Reihenfolge der Zudosierung der Medikamente lässt sich bisher nur schwer sicherstellen. Gleiche Probleme bestehen bei einer Anwendung durch den Patienten selbst, z.B. bei einer häuslicher Anwendung.

Aufgabe der vorliegenden Erfindung ist es daher, eine Klemme sowie ein System von Klemmen bereitzustellen, die eine sichere Lagerung und Zuordnung von insbesondere befüllten Spritzen unterschiedlicher Größen ermöglichen.

Diese Aufgabe wird erfindungsgemäß durch eine Klemme mit den Merkmalen des Anspruchs 1 gelöst. Eine solche Klemme weist vorzugsweise einen Grundkörper, der an zwei einander gegenüberliegenden Seiten aufeinander abgestimmte Verbindungselemente zur insbesondere lösbaren Verbindung gleichartiger Klemmen aufweist, und einen Aufnahmebereich auf, der zur insbesondere lösbaren Rastverbindung eines zylindrischen Gegenstands, beispielsweise einer Spritze, an dem Grundkörper im Wesentlichen ringsegmentartig und mit einer Aufnahmeöffnung ausgebildet ist, die sich bevorzugt auf weniger als 180° des Aufnahmebereichs erstreckt. Diese Dimensionierung der Aufnahmeöffnung des maulartigen Aufnahmebereichs ermöglicht die rasche und sichere Befestigung einer Spritze oder dergleichen in der Klemme bevorzugt durch eine Rast- bzw. Schnappverbindung. Erfindungsgemäß ist der Innendurchmesser des Aufnahmebereichs an den Außendurchmesser einer Spritze angepasst. Die Innendurchmesser des Aufnahmebereichs können beispielsweise von 10 mm für kleinvolumige Spritzen und bis zu 32 mm für großvolumige Spritzen betragen. Weiter wird es bevorzugt, wenn die einzelnen Klemmen unmittelbar über deren Verbindungselemente miteinander verbindbar sind.

Grundsätzlich kann bei einer erfindungsgemäßen Klemme die Aufnahmeöffnung des Aufnahmebereichs sehr klein gehalten werden bzw. auf diese sogar vollständig verzichtet werden, so dass der Aufnahmebereich als ein im Wesentlichen oder vollständig geschlossener Ring ausgebildet ist. Eine Spritze muss dann in Richtung der Längsachse in diesen ringförmigen Aufnahmebereich eingesteckt werden.

Die erfindungsgemäße Klemme sowie das unten näher beschriebene System stellen eine einfache und sichere Zubereitungshilfe für mehrere Arzneistoffe dar und ermöglichen eine einfach handhabbare Darreichungsform hierfür. Insbesondere wird die Zubereitung beispielsweise in einer Apotheke oder im Krankenhaus erheblich erleichtert, da eine schnelle und einfache Verbindung von Spritzen mit einer Hahnbank (beispielsweise erhältlich von Fresenius Kabi unter dem Namen "Hahnbank 5-fach" mit der Bestellnummer 8501342) möglich ist. So kann auch die Reihenfolge der Zudosierung von Medikamenten in Fällen der Anwendung durch den Patienten bspw. durch entsprechende farbliche Markierung der Hähne der Hahnbank einfacher beschrieben werden.

Die erfindungsgemäße Klemme wird vorzugsweise als ein Einwegteil verwendet, das nach der Benutzung verworfen wird. Es wird daher eine kostengünstige Herstellung, beispielsweise als ein einstückiges Spritzgussteil aus Kunststoff bevorzugt. Grundsätzlich kann eine erfindungsgemäße Klemme auch aus anderen geeigneten Materialien bestehen, bspw. aus Metall. Um bei der Verwendung mehrerer Klemmen in Kombination mit einer Hahnbank sicherzustellen, dass die Auslassstutzen der Spritzen unabhängig von deren Durchmesser in einer gemeinsamen Ebene und in einem durch die Einlassstutzen der Hahnbank vorgegebenen gleichmäßigen Abstand angeordnet sind, kann der Aufnahmebereich unmittelbar in den Grundkörper integriert sein, auf diesem angeordnet sein oder unter Zwischenschaltung eines Abstandshalters mit diesem verbunden sein.

Die der Erfindung zugrunde liegende Aufgabe wird weiter mit einem System nach Anspruch 3 gelöst. In einem erfindungsgemäßen System aus mehreren Klemmen, beispielsweise der oben genannten Art, von denen sich wenigstens zwei Klemmen voneinander unterscheiden, verfügen alle Klemmen über einen zumindest hinsichtlich seiner Abmessungen in Breitenrichtung identischen Grundkörper, der an zwei einander gegenüberliegenden Seiten aufeinander abgestimmte Verbindungselemente zur Verbindung mit weiteren Klemmen des Systems aufweist. Mit anderen Worten lassen sich Klemmen zur Aufnahme unterschiedlich großer Spritzen nicht nur untereinander verbinden, sondern die Breite der miteinander verbundenen Klemmen des Systems ist unabhängig von der Größe der in dieser aufgenommenen Spritzen gleich und dabei vorzugsweise an die Abstände von Einlassstutzen einer Hahnbank angepasst. Weiter weisen die Klemmen des Systems ggf. einen Abstandshalter und einen Aufnahmebereich auf, der zur Verbindung mit einer Spritze oder dergleichen zylindrischen Gegenstand z.B. ringsegmentartig ausgebildet ist und eine Längsachse definiert. Diese Längsachse des Aufnahmebereichs fällt bei Spritzen mit zentralem Auslassstutzen mit der Längsachse dieses Auslassstutzens zusammen. Erfindungsgemäß sind die Grundkörper und Aufnahmebereiche der Klemmen derart gestaltet, dass bei drei miteinander verbundenen Klemmen, die sich durch verschiedenen Innendurchmesser ihrer Aufnahmebereiche unterscheiden können, deren Längsachsen in einer gemeinsamen Ebene und/oder in identischem Abstand voneinander liegen. Damit ist es möglich, Spritzen gleich welcher Größe in einer definierten Reihenfolge in Klemmen des erfindungsgemä-βen Systems für Lagerung und Transport zu befestigen, so dass die Spritzen in ihrem in den Klemmen aufgenommenen Zustand unmittelbar an einer Hahnbank angeschlossen werden können.

Bei einem erfindungsgemäßen System aus wenigstens zwei verschiedenen Klemmen ist bei wenigstens einer dieser Klemmen der Aufnahmebereich mit einem Innendurchmesser von wahlweise etwa 10 mm, ca. 17 mm, ca. 24 mm und/oder von etwa 32 mm ausgebildet. Dies erlaubt die sichere und rasche Befestigung von herkömmlichen Einwegspritzen in Klemmen des Systems.

Zur Anbindung an eine Hahnbank wird bevorzugt, wenn die Grundkörper und Aufnahmebereiche der Klemmen derart gestaltet sind, dass bei zwei verschiedenen miteinander verbundenen Klemmen deren Längsachsen in einem Abstand von etwa 36 mm zueinander liegen.

Die bspw. als Rastelemente dienenden Aufnahmebereiche der Klemmen haben vorzugsweise die gleiche Ausdehnung in Richtung ihrer Längsachsen.

Die Erfindung betrifft weiter eine Kombination aus wenigstens einer Klemme oder einem System der oben genannten Art und einer Hahnbank mit wenigstens drei, jeweils über einen Absperrhahn verschließbaren Anschlussstutzen zum Ankoppeln an eine Spritze, wobei alle Anschlussstutzen in einem gemeinsamen Sammelkanal mit einem Auslassanschluss münden. Dabei wird es erfindungsgemäß bevorzugt, wenn die Abstände der Anschlussstutzen der Hahnbank und die Klemmen des Systems derart aufeinander abgestimmt sind, dass unterschiedliche Spritzengrößen, die in untereinander befestigten Klemmen des Systems aufgenommen sind, unmittelbar an die Hahnbank anschließbar sind. Vorzugsweise ist wenigstens eine Klemme mit der Hahnbank direkt oder indirekt, bspw. mittels einer Spritze, verbunden.

Die Erfindung betrifft zudem die Verwendung einer Klemme bzw. eines Systems der eingangs genannten Art zum Transport und/oder zur Lagerung von in Spritzen aufgenommenen Medikamenten, insbesondere von Komponenten einer nicht lagerstabilen Medikamentenmischung, beispielsweise einem Chemotherapeutikum oder einem Schmerztherapeutikum. Besonders bevorzugt wird die Verwendung für Heimanwendungen mehrerer Arzneistoffe die in einer bestimmten Reihenfolge und/oder in einem bestimmten Mischungsverhältnis verabreicht und/oder zubereitet werden müssen.

Nach einer weiteren Ausführungsform der Erfindung ist zusätzlich ein Rückschlagventil zwischen Spritze und dem 3-Wegestück der Hahnbank integriert oder vorgesehen, um ein unbeabsichtigtes Rückdrücken von Flüssigkeiten in eine der Spritzen zu vermeiden.

Die Erfindung wird nachfolgend anhand eines Ausführungsbeispiels und unter Bezugnahme auf die Zeichnung näher erläutert. Es zeigen schematisch:
- Fig. 1: eine perspektivische Draufsicht auf zwei erfindungsgemäße Klemmen,
- Fig. 2: die Klemmen nach Fig. 1 in Perspektivansicht von unten
- Fig. 3: ein erfindungsgemäßes System aus mehreren Klemmen und
- Fig. 4: ein erfindungsgemäßes System aus mehreren mit einer Hahnbank verbundenen Klemmen.

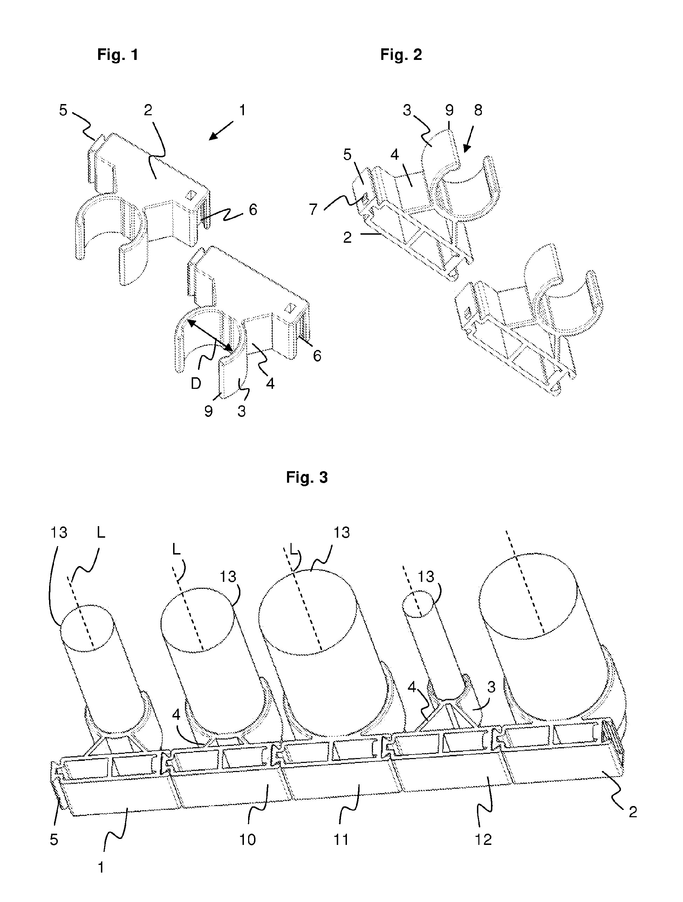

Die in den Fig. 1 und 2 dargestellten Klemmen 1 bestehen im Wesentlichen aus einem Grundkörper 2 und einem maulartigen Aufnahmebereich 3, der in der dargestellten Ausführungsform über einen Abstandshalter einstückig mit dem Grundkörper 2 verbunden ist. Die gesamte Klemme 1 ist als ein Spritzgussteil aus Kunststoff hergestellt.

Der Grundkörper 2 ist auf seiner in Fig. 1 rechten Seite mit einem schwalbenschwanzartigen Vorsprung 5 und auf seiner gegenüberliegenden Seite mit einer entsprechend konturierten Nut 6 versehen, die gemeinsam aufeinander abgestimmte Verbindungselemente zur Verbindung zweier Klemmen 1 bilden. Alternativ zu der in dem Ausführungsbeispiel der Fig. 1 und 2 dargestellten Vorsprung 5 und der entsprechenden Nut 6 können auch andere Querschnitte, beispielsweise ein etwa pilzkopfförmiger Vorsprung oder ein T-förmiger Vorsprung und eine entsprechend gestaltete Nut vorgesehen werden, die eine dauerhafte oder lösbare Verbindung zweier Klemmen ermöglichen.

Um ein ungewolltes Lösen der Verbindung zweier Klemmen 1 während des Transports zu verhindern oder zu erschweren, ist in der dargestellten Ausführungsform eine Rastnase 7 an dem Vorsprung 5 ausgebildet, die beim Verbinden zweier Klemmen 1 in eine entsprechende Ausnehmung (nicht gezeigt) in der Nut 6 einrasten kann.

Der Aufnahmebereich 3 der Klemme 1 ist im Wesentlichen ringsegmentartig ausgebildet, wobei zwischen den beiden gekrümmten Schenkeln des Aufnahmebereichs eine Aufnahmeöffnung 8 verbleibt, die sich auf weniger als 180° des Aufnahmebereichs 3 erstreckt. Mit anderen Worten kann der Aufnahmebereich 3 mit seinen beiden Schenkeln einen zylindrischen Gegenstand, wie eine Spritze, um mehr als 180° im Sinne einer Rast- oder Schnappverbindung umgreifen. Hierzu können die beiden Schenkel des Aufnahmebereichs 3 durch entsprechend Materialwahl und/oder konstruktive Mittel federnd gestaltet sein. In der dargestellten Ausführungsform sind zudem die dem Grundkörper 2 abgewandten Enden der beiden Schenkel des Aufnahmebereichs 3 mit einer wulstartigen Verdickung 9 versehen, die das Einrasten einer Spritze oder dergleichen erleichtert bzw. das ungewollte Herausfallen einer Spritze aus der Klemme 1 erschwert.

Der Aufnahmebereich 3 umschließt teilweise einen zumindest im Wesentlichen zylindrischen Raum, dessen durch die Schenkel des Aufnahmebereichs 3 begrenzter Innendurchmesser D an die Außenabmessungen einer Spritze angepasst ist. So kann der Innendurchmesser D beispielsweise etwa 10 mm bis 32 mm betragen.

In Fig. 3 ist ein erfindungsgemäßes System aus mehreren Klemmen 1, 10, 11 und 12 dargestellt. Die Gestaltung aller Klemmen dieses Systems ist im Wesentlichen gleich, wobei insbesondere der Grundkörper 2 jeweils im Wesentlichen identisch gestaltet ist. Die Klemmen 1, 10, 11 und 12 unterscheiden sich jedoch insbesondere hinsichtlich des Innendurchmessers D, der durch die Schenkel der jeweiligen Aufnahmebereiche 3 definiert wird. So eignen sich die Klemmen 1, 10, 11 und 12 zur Aufnahme und Befestigung unterschiedlich großer Spritzen 13, die in Fig. 3 durch Zylinder schematisch angedeutet sind.

Zudem unterscheiden sich die Klemmen 1, 10, 11 und 12 hinsichtlich der Größe des Abstandshalters 4 voneinander. So ist bei der Klemme 11 zur Aufnahme einer großen Spritze der Abstandshalter vollständig entfallen, während der Abstandshalter 4 bei der Klemme 12 zur Aufnahme einer Spritze mit kleinem Durchmesser besonders groß ausfällt. Grundsätzlich wäre es auch möglich, abweichend von den dargestellten Ausführungsformen für besonders große Spritzen den Aufnahmebereich 3 zumindest teilweise in den Grundkörper 2 zu integrieren.

Die Klemmen 1, 10, 11 und 12 sind als Bestandteile eines Systems derart aneinander angepasst, dass sie in beliebiger Reihenfolge über jeweils baugleiche Verbindungselemente, d.h. Vorsprünge 5 und Nuten 6, aneinander befestigbar sind. Die Grundkörper 2, die Aufnahmebereiche 3 und ggf. die Abstandshalter 4 sind dabei so ausgelegt, dass die Längsachsen L, die durch die jeweiligen Schenkel der Aufnahmebereiche 3 definiert werden, und die mit den Längsachsen der in den Klemmen aufgenommenen Spritzen 13 zusammenfallen, unabhängig von der Größe der Spritze 13 in einer gemeinsamen Ebene liegen und den gleichen Abstand voneinander aufweisen. Mit anderen Worten ist der Freiraum zwischen zwei jeweils in einer Klemme 11 aufgenommenen großen Spritzen kleiner als der Freiraum zwischen zwei jeweils in einer Klemme 12 aufgenommenen kleinen Spritzen. Durch die Anordnung der Längsachsen L in einer gemeinsamen Ebene und in gleichem Abstand voneinander unabhängig von der Spritzengröße ist es möglich, auch verschieden große Spritzen, die in mehreren miteinander verbundenen Klemmen 1, 10, 11 und/oder 12 eingerastet sind, an eine Hahnbank (nicht dargestellt) anzuschließen, deren Anschlussstutzen in einem definierten Abstand voneinander und in einer gemeinsamen Ebene liegen.

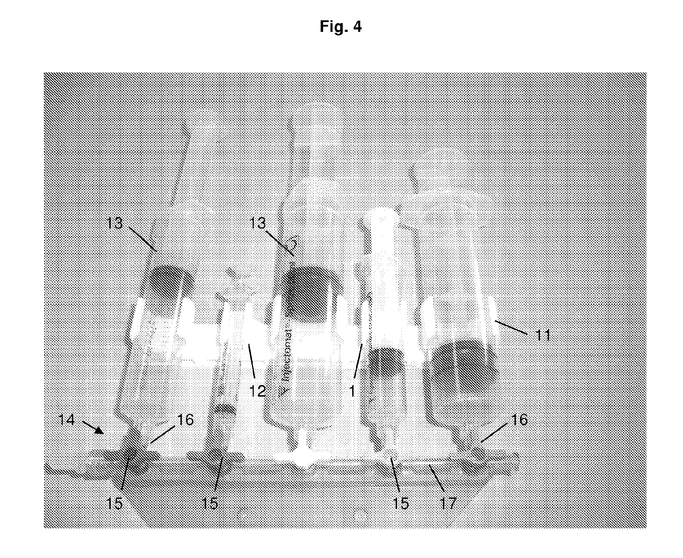

In Fig. 4 ist eine Hahnbank 14 dargestellt, die mehrere Absperrhähne aufweist, die in der gezeigten Ausführungsform als 3-Wege-Hähne 15 ausgebildet sind. Die Absperrhähne 15 sind jeweils einem Anschlussstutzen 16 zugeordnet, die wiederum in einen gemeinsamen Sammelkanal 17 mit einem Auslassanschluss münden. In den untereinander verbundenen Klemmen 1, 10, 11, 12 sind Spritzen 13 aufgenommen, die jeweils mit einem Anschlussstutzen 16 verbunden sind. Auf diese Weise sind die Klemmen über die Spritzen 13 mit der Hahnbank 14 verbunden. Abweichend von der dargestellten Ausführungsform kann auch eine direkte Verbindung zwischen der Hahnbank und den Klemmen vorgesehen sein.

Grundsätzlich ist es abweichend von der Darstellung in den Figuren auch möglich, auf eine Aufnahmeöffnung 8 des Aufnahmebereichs 3 vollständig zu verzichten, so dass die Aufnahmebereiche als ein geschlossener Ring ausgebildet sind. Eine Spritze muss dann in Richtung der Längsachse L in diesen ringförmigen Aufnahmebereich eingesteckt werden. Es wird jedoch die in den Figuren dargestellte Ausführungsform bevorzugt, die eine Rast- oder Schnappverbindung mit Spritzen oder dergleichen ermöglicht.

### Bezugszeichenliste:

- 1: Klemme
- 2: Grundkörper
- 3: Aufnahmebereich
- 4: Abstandshalter
- 5: Vorsprung
- 6: Nut
- 7: Rastnase
- 8: Aufnahmeöffnung
- 9: Verdickung
- 10: Klemme
- 11: Klemme
- 12: Klemme
- 13: Spritze
- 14: Hahnbank
- 15: Absperrhahn
- 16: Anschlussstutzen
- 17: Sammelkanal

- D: Innendurchmesser
- L: Längsachse

## Patentansprüche

1. Klemme zur Aufnahme von zylindrischen Gegenständen mit einem Grundkörper (2), der an zwei einander gegenüberliegenden Seiten aufeinander abgestimmte Verbindungselemente (5, 6) zur lösbaren Verbindung gleichartiger Klemmen (1, 10, 11, 12) aufweist, und einem Aufnahmebereich (3), der zur lösbaren Rastverbindung eines zylindrischen Gegenstands (13) an dem Grundkörper (2) im Wesentlichen ringsegmentartig und mit einer Aufnahmeöffnung (8) ausgebildet ist, die sich auf weniger als 180° des Aufnahmebereichs (3) erstreckt, wobei der Innendurchmesser (D) des Aufnahmebereichs (3) 32 mm, 17 mm bis 24 mm oder 10 mm beträgt.

2. Klemme nach Anspruch 1, **dadurch gekennzeichnet, dass** der Aufnahmebereich (3) mit dem Grundkörper (2) einstückig, ggf. unter Zwischenschaltung eines Abstandshalters (4), verbunden ist.

3. System aus wenigstens drei Klemmen (1, 10, 11, 12), insbesondere nach einem der Ansprüche 1 oder 2, wobei alle Klemmen (1, 10, 11, 12) einen zumindest im Wesentlichen identischen Grundkörper (2), der an zwei einander gegenüberliegenden Seiten aufeinander abgestimmte Verbindungselemente (5, 6) zur Verbindung mit weiteren Klemmen des Systems aufweist, ggf. einen Abstandshalter (4) und einen Aufnahmebereich (3) aufweisen, der zur Verbindung mit einem zylindrischen Gegenstand (13) im Wesentlichen ringförmig oder ringsegmentartig ausgebildet ist und eine Längsachse (L) definiert, **dadurch gekennzeichnet, dass** die Grundkörper (2) und Aufnahmebereiche (3) der Klemmen (1, 10, 11, 12) derart gestaltet sind, dass bei drei miteinander verbundenen Klemmen (1, 10, 11, 12), die sich durch verschiedene Innendurchmesser (D) ihrer Aufnahmebereiche (3) unterscheiden, deren Längsachsen (L) in einer gemeinsamen Ebene und/oder in einem identischen Abstand voneinander liegen.

4. System nach Anspruch 3 aus wenigstens zwei verschiedenen Klemmen (1, 10, 11, 12), deren Aufnahmebereiche (3) einen Innendurchmesser (D) von etwa 32 mm, etwa 17 mm bis etwa 24 mm oder etwa 10 mm aufweist.

5. System nach Anspruch 3 oder 4, **dadurch gekennzeichnet, dass** die Grundkörper (2) und Aufnahmebereiche (3) der Klemmen (1, 10, 11, 12) derart gestaltet sind, dass bei zwei verschiedenen miteinander verbundenen Klemmen deren Längsachsen (L) in einem Abstand von etwa 36 mm zueinander liegen.

6. System nach einem der Ansprüche 3 bis 5, **dadurch gekennzeichnet, dass** der Aufnahmebereich (3) wenigstens einer der Klemmen (1, 10, 11, 12) als ein geschlossener Ring ausgebildet ist, in welchen ein zylindrischer Gegenstand einsteckbar ist.

7. System nach einem der Ansprüche 3 bis 6, **dadurch gekennzeichnet, dass** die Aufnahmebereiche (3), in Richtung ihrer Längsachsen (L) die gleiche Ausdehnung haben.

8. Kombination aus wenigstens einer Klemme nach einem der Ansprüche 1 oder 2 und einer Hahnbank (14) mit wenigstens drei, jeweils über einen Absperrhahn (15) verschließbaren Anschlussstutzen zum Ankoppeln an eine Spritze (13), wobei alle Anschlussstutzen (16) in einen gemeinsamen Sammelkanal (17) mit einem Auslassanschluss münden.

9. Kombination nach Anspruch 8, **dadurch gekennzeichnet, dass** wenigstens eine Klemme (1, 10, 11, 12) mit der Hahnbank (14) verbunden ist.

10. Verwendung einer Klemme (1, 10, 11, 12) nach einem der Ansprüche 1 oder 2 oder eines Systems nach einem der Ansprüche 3 bis 7 zum Transport und/oder zur Lagerung von in Spritzen (13) aufgenommenen Medikamenten.

11. Verwendung nach Anspruch 10 zum Transport und/oder zur Lagerung von jeweils in einer Spritze (13) aufgenommenen Komponenten einer nicht lagerstabilen Medikamentenmischung.

12. Verwendung nach Anspruch 10 oder 11 zum Transport und/oder zur Lagerung von in Spritzen (13) aufgenommenen Chemotherapeutika oder Schmerztherapeutika.

## Geänderte Patentansprüche

### Geänderte Patentansprüche gemäss Regel 137(2) EPÜ.

**1.** System aus wenigstens drei Klemmen (1, 10, 11, 12), wobei alle Klemmen (1, 10, 11, 12) einen zumindest hinsichtlich seiner Abmessungen in Breitenrichtung identischen Grundkörper (2), der an zwei einander gegenüberliegenden Seiten aufeinander abgestimmte Verbindungselemente (5, 6) zur Verbindung mit weiteren Klemmen des Systems aufweist, ggf. einen Abstandshalter (4) und einen Aufnahmebereich (3) aufweisen, der zur Verbindung mit einem zylindrischen Gegenstand (13) im Wesentlichen ringsegmentartig ausgebildet ist und eine Längsachse (L) definiert, **dadurch gekennzeichnet, dass** die Grundkörper (2), Abstandshalter (4) und Aufnahmebereiche (3) der Klemmen (1, 10, 11, 12) derart gestaltet sind, dass bei drei miteinander verbundenen Klemmen (1, 10, 11, 12), von denen sich wenigstens zwei durch verschiedene Innendurchmesser (D) ihrer Aufnahmebereiche (3) unterscheiden, deren Längsachsen (L) in einer gemeinsamen Ebene liegen.

**2.** System nach Anspruch 1, wobei der Aufnahmebereich (3) mit einer Aufnahmeöffnung (8) ausgebildet ist, die sich auf weniger als 180° des Aufnahmebereichs (3) erstreckt.

**3.** System nach Anspruch 1 oder 2, wobei der Aufnahmebereich (3) mit dem Grundkörper (2) einstückig, ggf. unter Zwischenschaltung eines Abstandshalters (4), verbunden ist.

**4.** System nach einem der vorstehenden Ansprüche, wobei der Abstandshalter (4) derart gestaltet ist, dass die Längsachsen (L) von Klemmen unterschiedlicher Größen in einer Ebene liegen.

**5.** System nach einem der vorstehenden Ansprüche, wobei die Längsachsen (L) in einem identischen Abstand voneinander liegen.

**6.** System nach einem der vorstehenden Ansprüche, deren Aufnahmebereich (3) einen Innendurchmesser (D) von etwa 32 mm, etwa 17 mm bis etwa 24 mm oder etwa 10 mm aufweist.

**7.** System nach einem der vorstehenden Ansprüche, **dadurch gekennzeichnet, dass** die Grundkörper (2) und Aufnahmebereiche (3) der Klemmen (1, 10, 11, 12) derart gestaltet sind, dass bei zwei verschiedenen miteinander verbundenen Klemmen deren Längsachsen (L) in einem Abstand von etwa 36 mm zueinander liegen.

**8.** System nach einem der vorstehenden Ansprüche, **dadurch gekennzeichnet, dass** der Aufnahmebereich (3) wenigstens einer der Klemmen (1, 10, 11, 12) als ein geschlossener Ring ausgebildet ist, in welchen ein zylindrischer Gegenstand einsteckbar ist.

**9.** System nach einem der vorstehenden Ansprüche, **dadurch gekennzeichnet, dass** die Aufnahmebereiche (3), in Richtung ihrer Längsachsen (L) die gleiche Ausdehnung haben.

**10.** Kombination aus einem System nach einem der vorstehenden Ansprüche und einer Hahnbank (14) mit wenigstens drei, jeweils über einen Absperrhahn (15) verschließbaren Anschlussstutzen zum Ankoppeln an eine Spritze (13), wobei alle Anschlussstutzen (16) in einen gemeinsamen Sammelkanal (17) mit einem Auslassanschluss münden.

**11.** Kombination nach Anspruch 10, **dadurch gekennzeichnet, dass** wenigstens eine Klemme (1, 10, 11, 12) mit der Hahnbank (14) verbunden ist.

**12.** Verwendung eines Systems nach einem der vorstehenden Ansprüche zum Transport und/oder zur Lagerung von in Spritzen (13) aufgenommenen Medikamenten.

**13.** Verwendung nach Anspruch 12 zum Transport und/oder zur Lagerung von jeweils in einer Spritze (13) aufgenommenen Komponenten einer nicht lagerstabilen Medikamentenmischung.

**14.** Verwendung nach Anspruch 12 oder 13 zum Transport und/oder zur Lagerung von in Spritzen (13) aufgenommenen Chemotherapeutika oder Schmerztherapeutika.
